# EUROPEAN PATENT SPECIFICATION

(11) **EP 4 010 461 B1**
(45) Date of publication and mention of the grant of the patent: **01.04.2026**
(21) Application number: 20849918.6
(22) Date of filing: 04.08.2020
(51) Int. Cl.: C07K 14/47, C07K 14/705, C07K 14/71, C07K 14/725, C07K 16/28, C07K 16/32, C12N 15/86, C12N 5/071, G01N 33/50, G01N 33/569

(54) **ARTIFICIAL TARGET CELLS FOR IN-VITRO CAR CYTOTOXICITY AND ADCC VALIDATION**
KÜNSTLICHE ZIELZELLEN FÜR IN-VITRO-CAR-ZYTOTOXIZITÄT UND ADCC-VALIDIERUNG
CELLULES CIBLES ARTIFICIELLES POUR LA CYTOTOXICITÉ DE CAR IN-VITRO ET LA VALIDATION D'ADCC

(30) Priority: 05.08.2019 US 201962882772 P
(43) Date of publication of application: 15.06.2022
(73) Proprietor: ImmunityBio, Inc., Culver City, CA 90232 (US)
(72) Inventor: KLINGEMANN, Hans G., San Diego, California 92121 (US); BOISSEL, Laurent H., San Diego, California 92121 (US); DANDAPAT, Abhijit, San Diego, California 92121 (US)
(74) Representative: Elkington and Fife LLP
(86) International application number: PCT/US2020/044888
(87) International publication number: WO 2021/026155

(56) References cited:
- WO-A1-2013/126813
- WO-A1-2014/134165
- WO-A1-2020/028656
- WO-A1-2021/154218
- WO-A2-2016/164731
- WANG LAN ET AL: "Development of a robust reporter gene assay to measure the bioactivity of anti-PD-1/anti-PD-L1 therapeutic antibodies", vol. 145, 1 October 2017 (2017-10-01), AMSTERDAM, NL, pages 447 - 453, XP093064541, ISSN: 0731-7085, Retrieved from the Internet <URL:https://pdf.sciencedirectassets.com/271442/1-s2.0-S0731708517X00101/1-s2.0-S0731708517301826/main.pdf?X-Amz-Security-Token=IQoJb3JpZ2luX2VjENX//////////wEaCXVzLWVhc3QtMSJHMEUCIQDGyteXv8mk/CcXcGuMnYKcz2pD6KOfpaRjLHQ1bTrIUwIgIROtTUL3JXSVrPXaanf9yG0NOIIXF2LZDkuNX7NZAvEqsgUIXhAFGgwwNTkwMDM1NDY4NjUiDEPB4> DOI: 10.1016/j.jpba.2017.05.011
- ROY L MAUTE ET AL: "Engineering high-affinity PD-1 variants for optimized immunotherapy and immuno-PET imaging", PROCEEDINGS OF THE NATIONAL ACADEMY OF SCIENCES, NATIONAL ACADEMY OF SCIENCES, vol. 112, no. 47, 1 November 2015 (2015-11-01), pages E6506 - E6514, XP002772779, ISSN: 0027-8424, DOI: 10.1073/PNAS.1519623112
- SHU-RU KUO ET AL: "Engineering a CD123xCD3 bispecific scFvimmunofusion for the treatment of leukemiaand elimination of leukemia stem cells", PROTEIN ENGINEERING, DESIGN AND SELECTION, OXFORD JOURNAL, LONDON, GB, vol. 25, no. 10, 1 October 2012 (2012-10-01), pages 561 - 569, XP002721301, ISSN: 1741-0126, [retrieved on 20120627], DOI: 10.1093/PROTEIN/GZS040
- MAADI HAMID ET AL: "The effects of trastuzumab on HER2-mediated cell signaling in CHO cells expressing human HER2", vol. 18, no. 1, 1 March 2018 (2018-03-01), XP093064410, Retrieved from the Internet <URL:https://bmccancer.biomedcentral.com/counter/pdf/10.1186/s12885-018-4143-x.pdf> DOI: 10.1186/s12885-018-4143-x
- CHRISTOPHE LALLEMAND ET AL: "A Novel System for the Quantification of the ADCC Activity of Therapeutic Antibodies", JOURNAL OF IMMUNOLOGY RESEARCH, vol. 2017, 1 January 2017 (2017-01-01), US, pages 1 - 19, XP055437885, ISSN: 2314-8861, DOI: 10.1155/2017/3908289
- SCHOMER NATHAN THOMAS ET AL: "Providing a Homing Receptor for CAR Engineered NK Cells - Improving Cellular Immunotherapy for B-Cell Lymphoma", BLOOD, AMERICAN SOCIETY OF HEMATOLOGY, US, vol. 132, 29 November 2018 (2018-11-29), pages 4547, XP086593850, ISSN: 0006-4971, DOI: 10.1182/BLOOD-2018-99-116890
- GUOQING WEI, ET AL: "Novel immunotherapies for adult patients with B-lineage acute lymphoblastic leukemia", JOURNAL OF HEMATOLOGY & ONCOLOGY, vol. 10, no. 1, 1 December 2017 (2017-12-01), XP055453779, DOI: 10.1186/s13045-017-0516-x
- IMHOF BEAT ALBERT, MATTHES THOMAS: "New treatment for non-Hodgkin B-cell lymphomas with a special focus on the impact of junctional adhesion molecules", SWISS MEDICAL WEEKLY, XP055777617, DOI: 10.4414/smw.2017.14487
- MULLER, T. ET AL.: "Expression of a CD20-specific chimeric antigen receptor enhances cytotoxic activity of NK cells and overcomes NK-resistance of lymphoma and leukemia cells", CANCER IMMUNOL IMMUNOTHER, vol. 57, 2008, pages 411 - 423, XP019586697

## Description

### Field of the Invention

The present disclosure is directed to recombinant cells, and methods to assess effector cell functionality against a target cell population, particularly as it relates to ADCC or CAR-mediated cytotoxicity or bispecific engager (e.g., BiKES)-mediated cytotoxicity against cancer cells.

### Background of the Invention

The background description includes information that may be useful in understanding the present disclosure. It is not an admission that any of the information provided herein is prior art or relevant to the presently claimed invention, or that any publication specifically or implicitly referenced is prior art.

Where a definition or use of a term in a reference is inconsistent or contrary to the definition of that term provided herein, the definition of that term provided herein applies and the definition of that term in the reference does not apply.

Immune therapy of various cancers using antibody dependent cellular cytotoxicity (ADCC) of various effector cells and genetically modified T cells expressing a chimeric antigen receptor (CAR) have garnered significant interest and momentum in the scientific and medical community, and an increasing number of reports suggest that such immune therapy may be amenable across a wide range of cancers. As a consequence, there is an increasing need to develop new antibodies and CARs to target new cancers. Unfortunately, there are only a limited number of cell lines available that can represent or simulate target cancer cells for the purpose of testing CARs or antibodies *in vitro.* Moreover, NK effector cells often exhibit a relatively high spontaneous cytotoxic activity *in vitro,* making a distinction between background killing and targeted killing (CAR-mediated or ADCC) difficult. In addition, CAR cytotoxicity and ADCC assays that use adherent target cell lines can provide in at least some cases inconsistent results.

In an effort to address the various difficulties associated with quantification of ADCC and CAR mediated cytotoxicity, a set of specific target cells were generated where a first number of cells is modified to overexpress CD20, a second number of cells do not express CD20 at all, and effector cells are modified with a reported gene construct as is described in WO 2018/065401. While improving at least some issues associated with signal-to-noise ratio, various difficulties nevertheless remain. For example, substantial effort is required to generate three cell lines. Moreover, the systems and methods described in the '504 publication observe expression or function of the reported gene rather than actual cytotoxicity.

In another approach, as described in WO 2007/082138, a label-free system measures an increase in impedance between electrodes on a non-conducting substrate that supports the growth of target cells in an assay medium. Upon addition of effector cells, a decrease in impedance is then indicative of ADCC function of the effector cells. A similar system is described elsewhere (Cytometry A. 2017 Oct;91(10):1021-1029) in which antibody dose response is measured using an impedance method. While requiring no modifications on the effector cells, such assay systems have limited efficacy when using suspension cells. Moreover, such systems are in most cases still subject to high background signals due to non-specific cell killing by the effector cells.

Wang et al., J Pharm Biomed Anal. 2017 Oct 25:145:447-45 describes the development and validation of a reporter gene assay consisting of two-cell systems to measure the bioactivity of the anti-PD-1/anti-PD-L1 monoclonal antibodies. Lallemand et al., J Immunol Res. 2017:2017:3908289, describes ADCC effector cells expressing the V-variant or F-variant of FcγRIIIa (CD16a) and firefly luciferase under the control of a chimeric promoter incorporating recognition sequences for the principal transcription factors involved in FcγRIIIa signal transduction, that together with target cells overexpressing a constant high level of the specific antigen recognized by rituximab, trastuzumab, cetuximab, infliximab, adalimumab, or etanercept, confer improved sensitivity, specificity, and dynamic range in an ADCC assay relative to effector cells expressing a NFAT-regulated reporter gene and wild-type target cells.

Thus, even though various systems and methods of *in vitro* determination of CAR and ADCC mediated cell killing are known in the art, all or almost all of them suffer from various disadvantages. Consequently, there is a need to provide improved systems and methods that can quantitate ADCC and CAR-mediated cell killing with improved distinction between background killing and the targeted killing. In addition, improved systems and methods that can quantitate ADCC and CAR-mediated cell killing are desirable that can be used with nonadherent cells.

### Summary of The Invention

The invention is set out in the appended claims.

The inventors have discovered compositions, methods, and modified cells that enable quantification of ADCC and CAR-mediated cell killing with an improved distinction between background killing and targeted killing. Moreover, contemplated cells, compositions, and methods are also suitable for use with cells in suspension.

In one aspect of the inventive subject matter, the inventors contemplate a recombinant target cell that includes a recombinant nucleic acid that comprises a sequence encoding a transmembrane antigen, wherein the sequence is operably coupled to a promoter to express the sequence that encodes the transmembrane antigen. The cell exhibits equal or less than 20%, or equal or less than 15% spontaneous lysis in the presence of a CAR-T or an NK cell at an effector cell to target cell ratio of 1 or less.

The transmembrane antigen is a tumor associated antigen, a tumor specific antigen, or a patient- and tumor specific antigen, which may be present on a solid tumor or a liquid tumor. For example, suitable antigens include PD-L1, CD33, CD123, HER-2, and CD20. Preferably, but not necessarily, the recombinant nucleic acid is a recombinant lentiviral expression vector, or a recombinant RNA.

The recombinant target cell is a leukemia cell, specifically the cell is the ALL cell SUP-B15.

It is further contemplated that the cell exhibits equal or less than 15% spontaneous lysis in the presence of CAR-T or NK cell at an effector cell to target cell ratio of 10 or less, and/or that the cell exhibits equal or less than 10% spontaneous lysis in the presence of CAR-T or NK cell at an effector cell to target cell ratio of 1 or less. Most typically, the effector cell is a CAR-T cell or an NK cell.

Therefore, in another aspect of the inventive subject matter, the inventors also contemplate a method of quantifying ADCC or CAR-mediated cell killing, wherein the method includes a step of providing or generating a recombinant target cell of the first aspect of the invention, and a further step of providing or generating a CAR-T or an NK cell. The recombinant target cell and the CAR-T or NK cell are then incubated together at a predetermined effector cell to target cell ratio, and in a further step, the ADCC or CAR-mediated cell killing is quantified.

In further contemplated embodiments, the recombinant target cell exhibits equal or less than 15% spontaneous lysis in the presence of CAR-T or NK cell at an effector cell to target cell ratio of 10 or less or the recombinant target cell exhibits equal or less than 10% spontaneous lysis in the presence of CAR-T or NK cell at an effector cell to target cell ratio of 1 or less. As noted earlier, the transmembrane antigen expressed by the recombinant target cell is a tumor associated antigen, a tumor specific antigen, or a patient- and tumor specific antigen *(e.g.,* PD-L1, CD33, CD123, Her-2, or CD20).

While not limiting to the inventive subject matter, it is contemplated that the step of incubating is performed while the recombinant target cell and the CAR-T or NK cell are in suspension, and/or that the step of quantifying the ADCC or CAR-mediated cell killing is performed using a flow cytometry based cytotoxicity or ADCC assay. As will be readily appreciated, contemplated methods are not only suitable for validation of targets on a target cell, but also for screening an antibody library against various antigens, and especially transmembrane antigens.

Various objects, features, aspects, and advantages will become more apparent from the following detailed description of preferred embodiments, along with the accompanying drawing in which like numerals represent like components.

### Brief Description of The Drawing

Fig.1 is a schematic illustration of an exemplary cloning process and recombinant cells prepared by the inventors.
Fig.2A shows exemplary results for PD-L1 expression in recombinant SupB15^{PD-L1+} target cells.
Fig.2B shows exemplary comparative results for cytotoxicity of NK cells lacking expression of an anti-PD L1 CAR and recombinant NK cells expressing an anti-PD L1 CAR using non-recombinant target cells expressing PD-L1.
Fig.2C shows exemplary comparative results for cytotoxicity of NK cells lacking expression of an anti-PD L1 CAR and recombinant NK cells expressing an anti-PD L1 CAR using recombinant SupB15^{PD-L1+} target cells.
Fig.3A shows exemplary results for CD33 expression in recombinant SupB15^{CD33+} target cells.
Fig.3B shows exemplary comparative results for cytotoxicity of NK cells lacking expression of an anti-CD33 CAR and recombinant NK cells expressing an anti-CD33 CAR using non-recombinant target cells expressing CD33.
Fig.3C shows exemplary comparative results for cytotoxicity of NK cells lacking expression of an anti-CD33 CAR and recombinant NK cells expressing an anti-CD33 CAR using recombinant SupB15^{CD33+} target cells.
Fig.4A shows exemplary results for CD123 expression in recombinant SupB15^{CD33+} target cells.
Fig.4B shows exemplary comparative results for cytotoxicity of NK cells lacking expression of an anti-CD123 CAR and recombinant NK cells expressing an anti-CD123 CAR using non-recombinant target cells expressing CD123.
Fig.4C shows exemplary comparative results for cytotoxicity of NK cells lacking expression of an anti-CD123 CAR and recombinant NK cells expressing an anti-CD123 CAR using recombinant SupB15^{CD123+} target cells.
Fig.5A shows exemplary results for HER-2 expression in recombinant SupB15^{HER2+} target cells.
Fig.5B shows exemplary comparative results for cytotoxicity of NK cells lacking expression of an anti-HER2 CAR and recombinant NK cells expressing an anti-HER2 CAR using non-recombinant target cells expressing HER-2.
Fig.5C shows exemplary comparative results for cytotoxicity of NK cells lacking expression of an anti-HER2 CAR and recombinant NK cells expressing an anti-HER2 CAR using recombinant SupB15^{HER2+} target cells.
Fig.6A shows exemplary results for CD20 expression in recombinant SupB15^{CD20+} target cells.
Fig.6B shows exemplary comparative results for ADCC of NK cells expressing CD16 in the presence of a CD20 target specific antibody (Herceptin) and a sham antibody (Rituximab) using non-recombinant target cells expressing CD20.
Fig.6C shows exemplary comparative results for ADCC of NK cells expressing CD16 in the presence of a CD20 target specific antibody (Herceptin) and a sham antibody (Rituximab) using recombinant SupB15^{CD20+} target cells.
Fig.7 shows exemplary results for high background killing of NK cells against CD19⁺ DoHH2 cells.
Fig.8 is an exemplary lentiviral vector construct suitable for recombinant expression of various antigens on artificial target cells presented herein.

### Detailed Description

The invention is set out in the appended claims.

For the clinical evaluation of cellular immunotherapy, it is generally necessary to analyze *in vitro* effector cell functionality against a target cell population. Unfortunately, appropriate target cell lines to test *in vitro* spontaneous lysis versus ADCC or CAR-mediated cytotoxicity by the therapeutic effector cells are often not available. Moreover, where target cell lines are available, non-specific (with respect to the target) background killing of the target cells by NK effector cells is often relatively high, making it difficult to find clear differences between the background killing and the ADCC or CAR-mediated killing. Similarly, *in vitro* CAR cytotoxicity and ADCC assays with adherent target cell lines are inconsistent in most, if not all cases. To compound difficulties even further, established cell lines may not at all be available for certain cancer types to test CAR functionality *in vitro.*

In an effort to overcome these impediments, the inventors have discovered cells and cell lines that exhibit significantly resistance to spontaneous lysis by a CAR-T or an NK cell, and that such cells and cell lines can be genetically modified to express one or more antigens of therapeutic interest. Viewed from another perspective, when such cells and cell lines were used, non-specific (with respect to the target antigen) background killing of the target cells by the effector cells *(e.g.,* NK cells, CAR-T cells, etc.) was reduced to a significant degree, while target-specific cytotoxicity was observed when these cells and cell lines expressed suitable antigens. Therefore, it should be appreciated that substantially improved quantitative analysis of ADCC or CAR-mediated cytotoxicity is enabled in the presence of numerous and diverse antigens, and especially membrane-bound and/or transmembrane antigens as is shown in more detail below.

Therefore, based on the numerous observations, the inventors contemplate a recombinant SUP-B15 leukemia target cell that comprises a recombinant nucleic acid having a sequence that encodes a transmembrane antigen in the context of a promoter that drives expression of the transmembrane antigen. The transmembrane antigen is a tumor associated antigen, a tumor specific antigen, or a patient- and tumor-specific antigen. The recombinant target cell exhibits equal or less than 20%, or equal or less than 15% spontaneous lysis in the presence of a CAR-T or an NK cell at an effector cell to target cell ratio of 1 or less. As will be readily appreciated, such cells are especially suitable for methods of quantifying ADCC or CAR-mediated cell killing in which recombinant target cells and effector cells *(e.g.,* CAR-T or an NK cells) are provided and incubated/cultivated at a desired effector cell to target cell ratio for a predetermined time. Subsequently, the ADCC or CAR-mediated cell killing is measured using any suitable method known in the art.

The recombinant target cell is an acute lymphoblastic leukemia cell, specifically a SUP-B15 cell, which is commercially available from various sources (*e.g*., ATCC CRL-1929 from American Type Culture Collection (ATCC), 10801 University Boulevard, Manassas, VA 20110 USA). As shown in more detail below, these cells exhibited spontaneous lysis of 10-15% or less by NK effector cells, even when transfected to express a recombinant protein. Moreover, SUP-B15 cells advantageously grow in suspension culture and can be easily transfected using various transfection systems. As such, it should be noted that contemplated systems and methods allow for simple production of artificial target cell lines for *in vitro* CAR cytotoxicity and ADCC validation. Indeed, it was unexpectedly observed that the so prepared recombinant artificial target cells were better target cells in comparison to natural cancer target cell line carrying the same target antigen as the recombinant target cell. Notably, the recombinant artificial target cells had decreased spontaneous lysis/non-specific killing and/or increased target-specific lysis. In this context it should be noted that the terms "spontaneous lysis" and "non-specific killing" with regard to target cells refer to cell death of the target cells that is not attributable to a target specific (*i.e*., specific to the recombinant target antigen expressed in the target cells) cell killing event, wherein the target specific cell killing event is ADCC (via an antibody binding the recombinant target antigen) and/or CAR-mediated cytotoxicity (via the CAR ectodomain binding the recombinant target antigen).

Therefore, it should be appreciated that contemplated cells, systems, and methods provide a platform for *in vitro* ADCC validation by engineering target cells with cancer specific antigens that yield reliable and robust *in vitro* assays for the validation of effector cells with therapeutic antibodies. In heretofore known systems, *in vitro* CAR cytotoxicity validation almost completely depends on the availability of established target cell lines that express a CAR specific antigen. In contrast, using the systems and methods presented herein allows validating any CAR cytotoxicity on engineered target cells even in absence of established cell line. Also, different CAR cytotoxicity assays can be validated on the same artificial target cells. Beneficially, the system and methods presented herein can be implemented using any known quantification methods, including optical methods, capacitive methods, and flow cytometry methods. As will also be readily appreciated, the methods contemplated herein can be employed to screen for suitable antibodies from an antibody library.

With respect to suitable cells it is generally noted that all SUP-B15 cells are deemed appropriate for use herein so long as such cells exhibit a sufficiently low spontaneous lysis in the presence of a CAR-T or an NK cell. For example, suitable cells include those that exhibits equal or less than 15%, or equal or less than 14%, or equal or less than 13%, or equal or less than 12%, or equal or less than 11%, or equal or less than 10%, or equal or less than 8%, or equal or less than 5% spontaneous lysis in the presence of a CAR-T or an NK cell. Most preferably, such spontaneous lysis is observed at an effector cell to target cell ratio of at least 1, or of at least 2, of at least 3, of at least 5, of at least 7, or of at least 10. Therefore, exemplary cells will exhibit equal or less than 15% spontaneous lysis in the presence of a CAR-T or an NK cell at an effector cell to target cell ratio of at least 1, or equal or less than 15% spontaneous lysis in the presence of CAR-T or NK cell at an effector cell to target cell ratio of at least 5, or equal or less than 15% spontaneous lysis in the presence of CAR-T or NK cell at an effector cell to target cell ratio of at least 10, or equal or less than 10% spontaneous lysis in the presence of CAR-T or NK cell at an effector cell to target cell ratio of at least 1, or equal or less than 10% spontaneous lysis in the presence of CAR-T or NK cell at an effector cell to target cell ratio of at least 5, or equal or less than 10% spontaneous lysis in the presence of CAR-T or NK cell at an effector cell to target cell ratio of at least 10.

It is further preferred that the SUP-B15 leukemia target cells can be grown in suspension culture. However, in other embodiments SUP-B15 leukemia cells are also deemed suitable that are grown in adherent cell culture. Moreover, it should be appreciated that the cells are preferably mammalian and most preferably human cells that most typically will not natively express the antigen against which ADCC or CAR-mediated cell killing is directed. Moreover, it is generally preferred that the target cells will not require specific growth factors such as cytokines. As will be readily appreciated, contemplated target cells can be primary mammalian cells, and more typically secondary or immortalized cells that can be grown from a stock culture (live or frozen).

It should further be noted that where the effector cells are NK cells, suitable target cells may express (natively or from a recombinant nucleic acid) one or more ligands for a member of the human Killer Immunoglobulin-like Receptor (KIR) family or the mouse Ly49 family, one or more ligands for CD94-NKG2C/E/H heterodimeric receptors, one or more ligands for NKG2D, one or more ligands for natural cytotoxicity receptors such as NKp30, NKp44, and NKp46, and one or more ligands for the nectin/nectin-like binding receptors DNAM-1/CD226 and CRTAM. Further contemplated ligands include those that bind to SLAM family receptors including 2B4/CD244, CRACC/SLAMF7, and NTB-A/SLAMF6, CD27, CD100/Semaphorin 4D, and CD 160. Moreover, it should be noted that these ligands may also be co-expressed with the target antigen to so increase the signal for CAR or ADCC mediated cell killing.

Likewise, where the effector cells are CAR T cells, suitable target cells may also express (natively or from a recombinant nucleic acid) one or more ligands for T cell activation, including CD112, and ligands for CD28, OX40, GITR, CD137, CD27, and/or HVEM. Conversely, contemplated assays may also include various inhibitors of immune checkpoint signaling to further enhance CAR or bispecific engager-mediated cell killing.

With respect to effector cells, various effector cells are generally contemplated, including NK cells, NKT cells and T cells, which may be fresh isolates, obtained from culture, and/or genetically modified. In some embodiments, the effector cells are NK cells, which may be patient derived for autologous use, or genetically modified NK cells that express CD16 (or a high affinity variant thereof) and/or an intracellularly retained cytokine such as IL-2 and/or IL-15. Of course, it should also be appreciated that the genetically modified NK cells may further include a CAR construct (*e.g*., 1^{st}, 2^{nd}, 3^{rd} generation CAR, or CAR with an FcεRIγ). In other embodiments, the effector cells are T cells, which are preferably CAR-T cells, and which may be autologous or heterologous. As noted before, the nature of the CAR may vary considerably, and suitable CARs include 1^{st}, 2^{nd}, and 3^{rd} generation CAR constructs.

The particular manner of transfecting the artificial target cells is not deemed critical to the inventive subject matter, and all known manners of generating recombinant target cells are deemed suitable for use herein. Indeed, the particular manner of transfection may be at least in part dictated by the particular type of cell and its susceptibility to one or more transfection methods. Therefore, contemplated methods include viral transfection, lipofection, electroporation, ballistic gene transfer, etc. Therefore, it should be recognized that the type and configuration of the recombinant nucleic acid may vary considerably, and contemplated types include RNA and DNA constructs (which may be linear or circular, and which may be part of a viral vector). However, it is generally preferred that the recombinant nucleic acid will include a (typically constitutive) promoter that drives expression of the gene encoding the desired antigen. Moreover, the promoter will be of sufficient strength to drive expression of the desired antigen in a manner that will produce sufficient quantities of antigen needed for ADCC or CAR-mediated cell killing. In addition, it should be appreciated that recombinant nucleic acid may encode in addition to the antigen further proteins. For example, suitable additional proteins include co-activating ligands (with respect to ADCC or CAR-mediated cell killing) and checkpoint inhibitors.

With respect to the expressed transmembrane tumor associated antigens, tumor specific antigens, or patient- and tumor-specific antigens (neoantigens), it should be recognized that all clinically relevant antigens of this sort are deemed suitable for use herein. Especially preferred antigens include antigens that are present on a solid or liquid tumor. For example, contemplated antigens include PD-L1, CD33, CD123, HER-2, or CD20 as further described in more detail below.

Detection and quantification of ADCC or CAR-mediated cell killing can be performed in numerous manners, and all known manners are deemed suitable for use herein. Therefore, quantification may include various flow cytometric, optical, radiometric, and/or capacitive methods, and the particular choice of method will at least in part depend on the type of test and preexisting equipment.

### Examples

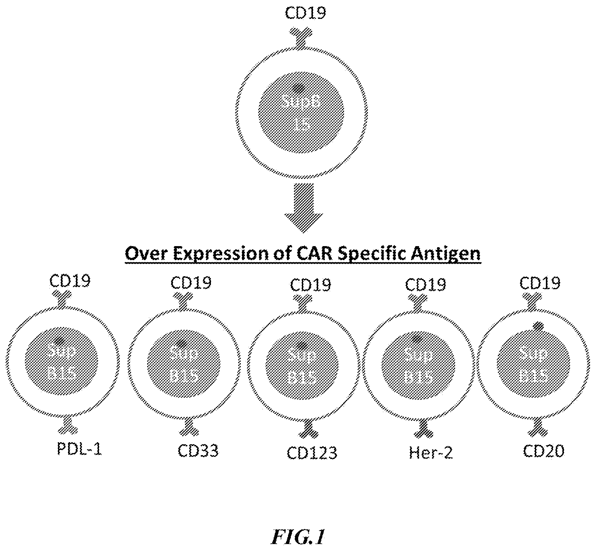

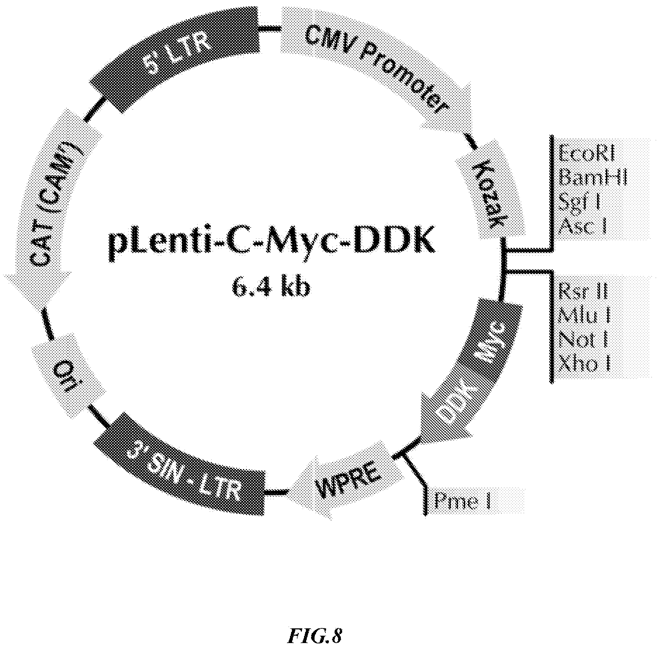

The following examples are provided as a general guidance and only to illustrate the inventive concept presented herein. Therefore, nothing in these examples should be construed as limiting the inventive subject matter. More particularly, the inventors generated various and distinct recombinant SUP-B15 cells that each expressed different transmembrane cancer associated or cancer specific antigens: CD33, PD-L1, CD123, HER-2 and CD20 as is schematically illustrated in **FIG.1****.** These antigens are generally not expressed on SUP-B15 lymphoma cells, and it should be appreciated that numerous alternative antigens may be expressed on these cells with similar results. Unless indicated otherwise, the lentiviral expression vector of **FIG.8** was used for recombinant expression of the exemplary antigens as noted below and NK cells were transfected with the recombinant lentiviral construct.

In this context it should be appreciated that the four cells lines SUP-B15^{CD33}, SUP-B15^{PD-L1}, SUP-B15^{CD123}, and SUP-B15^{HER-2} are mainly considered for *in vitro* CAR cytotoxicity quantification/validation, while the SUP-B15^{CD20} line would be more useful for *in-vitro* ADCC quantification/validation or assay development *(e.g.,* to identify suitable antibodies against CD20). It should also be recognized that until to date, there are no ideal (artificial) target cell lines for flow cytometry based *in-vitro* ADCC assay validation. Consequently, the SUP-B15^{CD20} cell line would be a particularly useful target cell line for flow cytometry based ADCC assay validation of haNK (NK cells expressing CD16 with high-affinity mutant at position 158) or t-haNK (NK cells expressing CD16 with high-affinity mutant at position 158 and expressing a CAR) cells in combination with Rituximab or other similarly targeting antibodies.

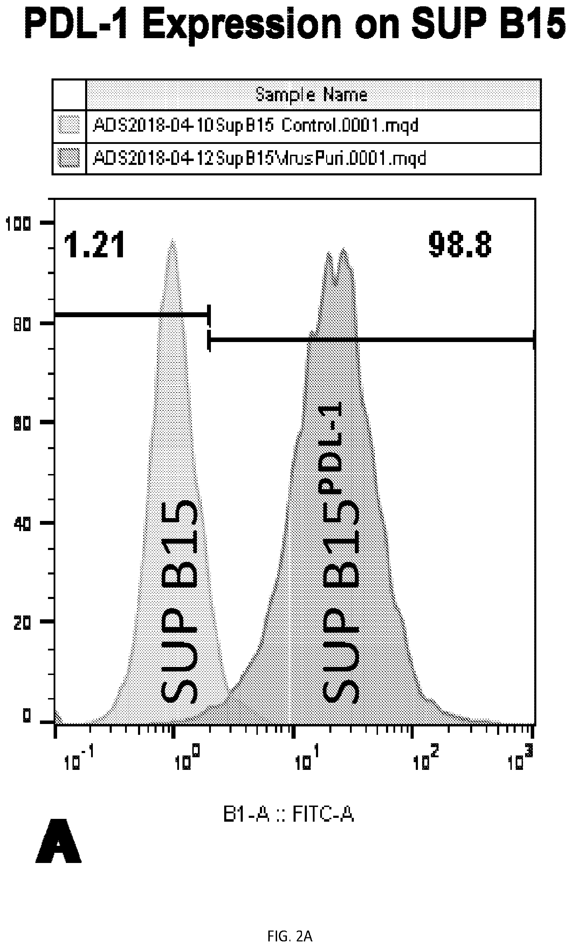

Example 1: PD-L1 (Programmed death-ligand 1) binding to PD-1 transmits an inhibitory signal that reduces the proliferation of antigen-specific T-cells in the tumor microenvironment. A PD-L1 expressing SUP-B15 line was generated by transduction with lentivirus particles carrying human PD-L1 cDNA having a cDNA sequence of SEQ ID NO:3. Surface expression of PD-L1 in SUP-B15 was validated by flow cytometry and exemplary results are shown in **FIG.2A****.** As can be readily seen from the graph, expression of PD L-1 on the SupB15 cells was strong and in a very large proportion of the cells.

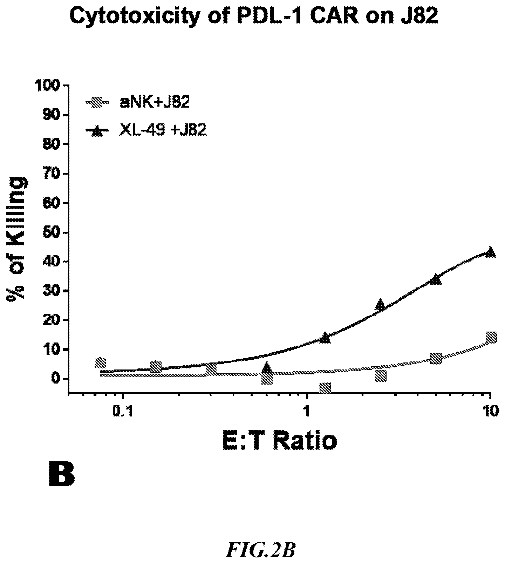

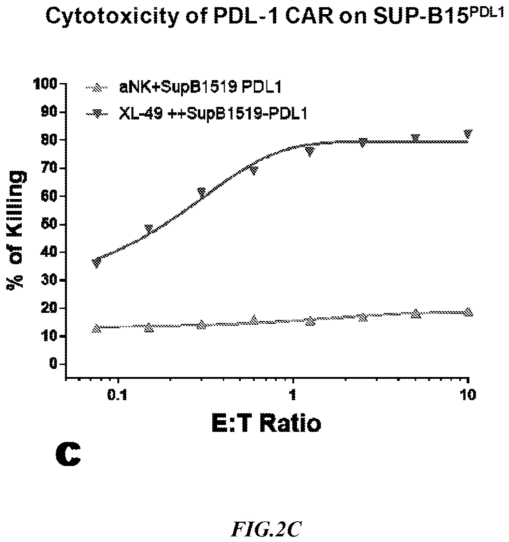

Cytotoxicity mediated by an anti-PD-L1 CAR expressing NK cell line (XL-49) was checked on SUP-B15^{PD-L1} artificial target cells and one naturally expressing PD-L1 target line, J82, side by side. Non-recombinant NK cells lacking expression of an anti-PD-L1 CAR (aNK) were used to detect non-specific cytotoxicity against the SUP-B15^{PD-L1} artificial target cells and against the target line J82 that naturally expressed PD-L1. Exemplary results are depicted in **FIG.2B** and **FIG.2C****.** As can be taken from the data, the PD-L1 CAR cytotoxicity at 1:1 ET (Effector: Target) on SUP-B15^{PDL-1} was 70% whereas the natural target, J82, showed only 10%. Clearly, the CAR cytotoxicity data indicated that the SUP-B15^{PDL-1} cell line is a far superior target than J82, a natural PD-L1 expressing target line. Moreover, due to the high killing rate with the artificial target cell line, the signal to background ratio (ratio of target-specific killing versus non-specific killing) was significantly improved. It should also be appreciated that the increase in non-specific killing at increased E:T ratios was substantially more pronounced with the J82 cells as compared to the artificial target cells.

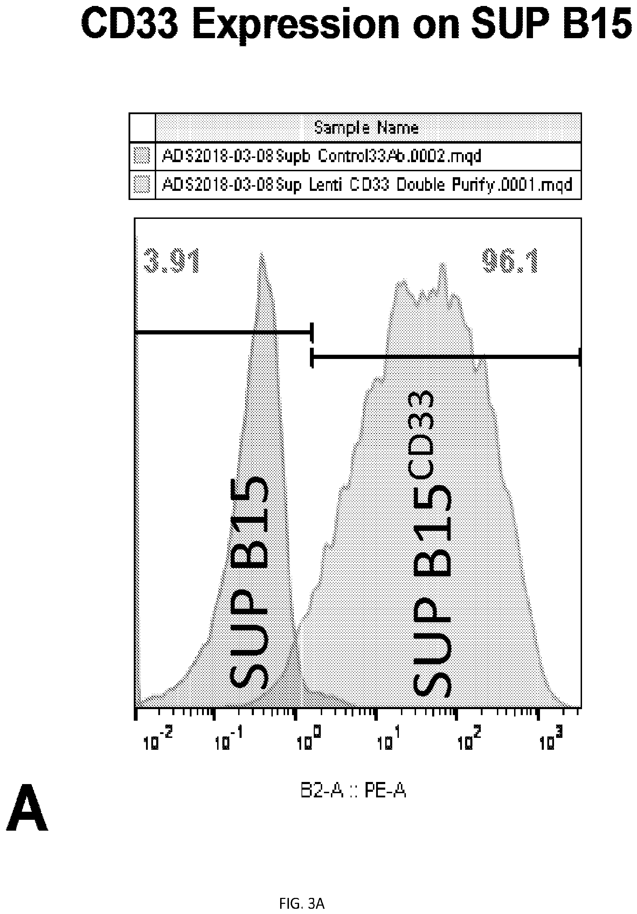

Example 2: CD33 is considered to be a marker of acute myeloid leukemia and is generally expressed on cells of myeloid lineage. SUP-B15 cells expressing CD33 were generated using a lentiviral vector carrying the human CD33 transcript variant 1 cDNA having SEQ ID NO:2 under puromycin selection. Surface expression of CD33 in SUP-B15 was validated by flow cytometry and typical results are shown in **FIG.3A****.** As will be readily appreciated, expression of CD33 on the SupB15 cells was strong and in a very large proportion of the cells.

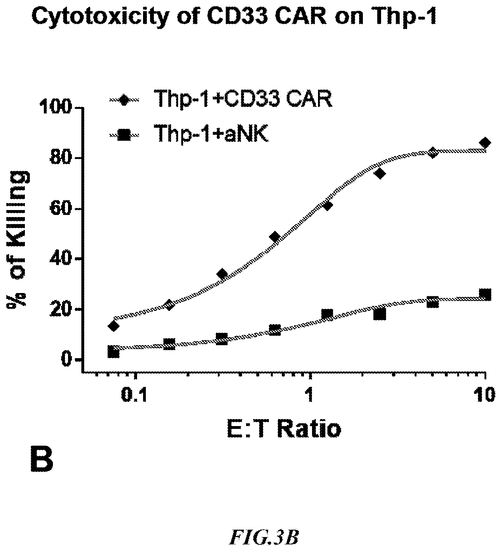

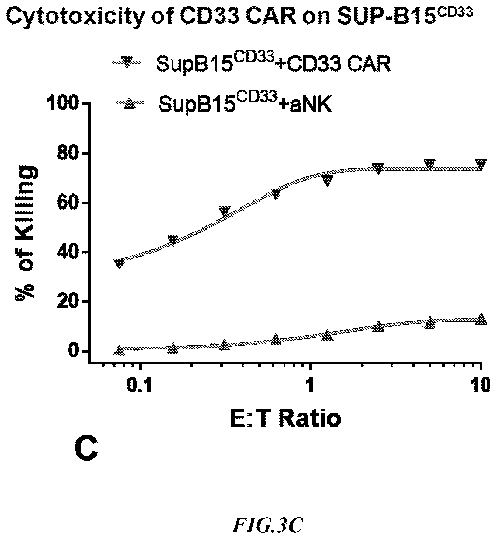

Experiments measuring the cytotoxicity mediated by an anti-CD33 CAR expressing NK cell using SUP-B15^{CD33} and the natural CD33-positive target line, THP-1, were run side by side. Non-recombinant NK cells lacking expression of an anti-CD33 CAR (aNK) were used to detect non-specific cytotoxicity against the SUP-B15^{CD33} artificial target cells and against the target line THP-1 that naturally expressed CD33. Exemplary results are shown in **FIG.3B** and **FIG.3C****.** Notably, the cytotoxicity of anti-CD33 CAR expressing NK cells at 1:1 E:T on SUP-B15^{CD33} cells was 70% whereas on THP-1 cells CAR cytotoxicity was only 45%. Moreover, non-specific background killing was substantially higher on THP-1 cells as compared to SUP-B15^{CD33} cells. It should also be appreciated that the increase in non-specific killing at increased E:T ratios was substantially more pronounced with the THP-1 cells as compared to the artificial target cells. These CD33 CAR cytotoxicity results indicate that the artificial target cells SUP-B15^{CD33} were a better target than the natural target THP-1 line in terms of target specific and non-specific cell killing.

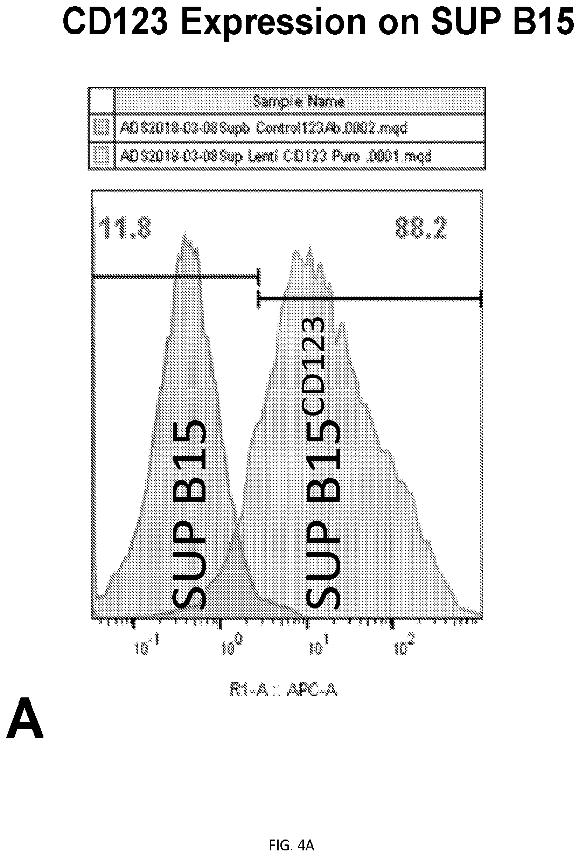

Example 3: CD123 is a membrane biomarker and a therapeutic target in hematologic malignancies. The SUP-B15^{CD123} line was generated by transduction with a lentiviral vector carrying the human interleukin 3 receptor alpha (CD123) cDNA having SEQ ID NO:1 under puromycin selection. Surface expression of CD123 in SUP-B15 cells was validated by flow cytometry as is shown in **FIG.4A****.** As will once more be readily appreciated, expression of CD123 on the SupB15 cells was strong and in a very large proportion of the cells.

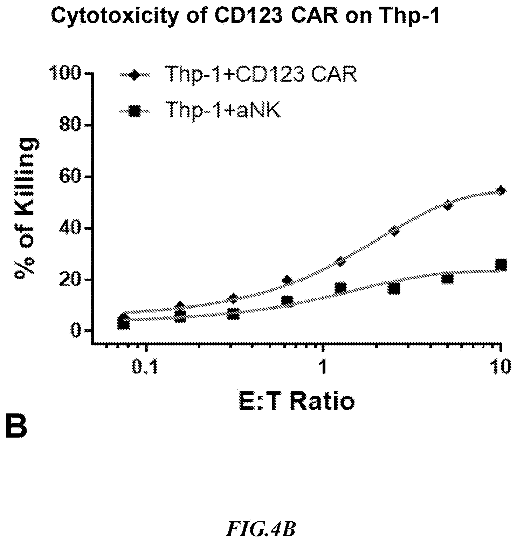

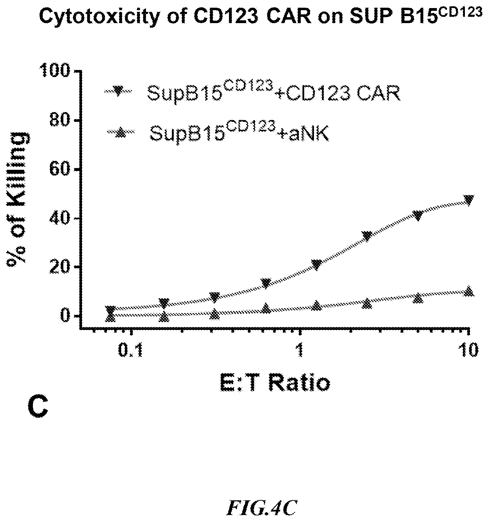

Experiments measuring the cytotoxicity mediated by an anti-CD123 CAR using SUP-B15^{CD123} and the natural CD123-positive target line, THP-1, were run side by side. Non-recombinant NK cells lacking expression of an anti-CD33 CAR (aNK) were used to detect non-specific cytotoxicity against the SUP-B15^{CD123} artificial target cells and against the target line THP-1 that naturally expressed CD123. Exemplary results are shown in **FIG.4B** and **FIG.4C****.** As can be taken from the results, the cytotoxicity of anti-CD123 CAR expressing NK cells at 1:1 E:T against both SUP-B15^{CD123} and THP-1 was at about 20%, but background killing was much higher with THP-1 cells. It should also be appreciated that the increase in non-specific killing at increased E:T ratios was substantially more pronounced with the THP-1 cells as compared to the artificial target cells. Considering CAR cytotoxicity and background killing data, the SUP-B15^{CD123} cell line is a significantly better target cell than the corresponding natural THP-1 line.

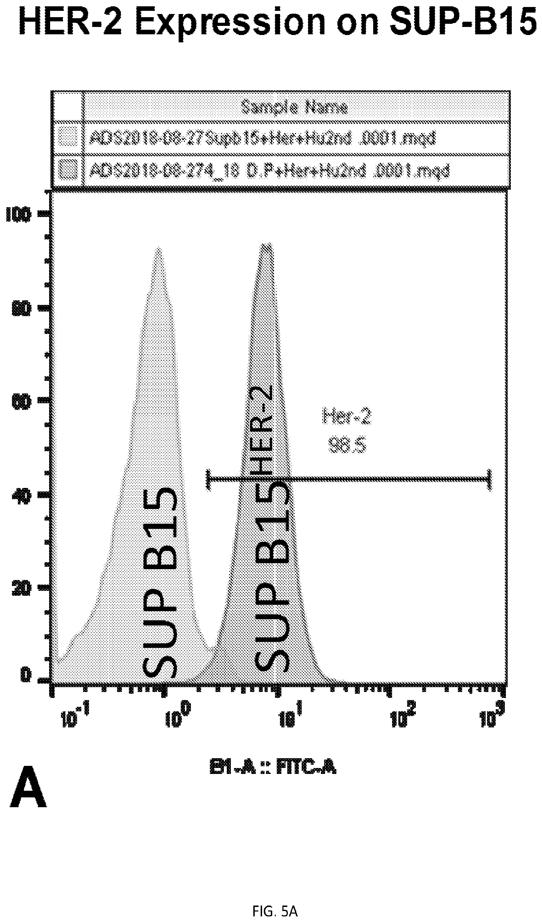

Example 4: HER2 (human epidermal growth factor receptor 2) is a gene that can play a role in the development of HER-2 positive breast cancer. A HER-2 overexpressing SUP-B15 cell line was generated by infecting the cells with a lentivirus comprising a human HER-2 cDNA having SEQ ID NO:4. Surface expression of HER2 in SUP-B15 was validated by flow cytometry as is depicted in the exemplary results of **FIG.5A****.** As can be readily appreciated, expression of HER2 on the SupB15 cells was strong and in a very large proportion of the cells

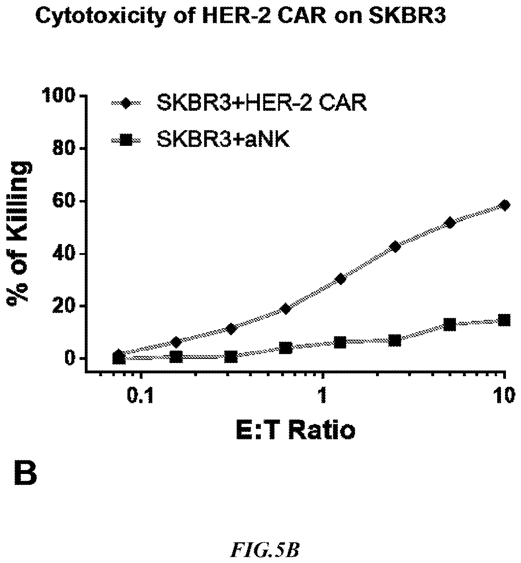

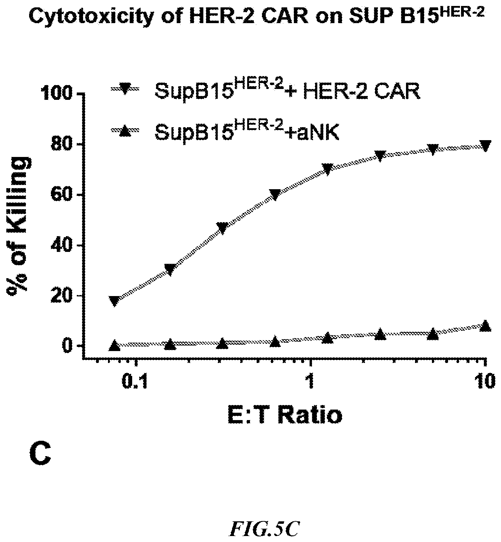

Cytotoxicity mediated by an anti-HER2 CAR expressing NK cell was determined on SUP-B15^{HER-2} and one of the natural target lines, SKBR3 expressing naturally HER2. Non-recombinant NK cells lacking expression of an anti-HER2 CAR (aNK) were used to detect non-specific cytotoxicity against the SUP-B15^{HER2} artificial target cells and against the target line SKBR3 that naturally expressed HER2. Exemplary results are shown side by side in **FIG.5B** and **FIG.5C****.** Notably, the HER-2 CAR cytotoxicity at 1:1 ET on SUP B15^{HER-2} was 60% whereas on SKBR3 it was 25%. Once more, it should be appreciated that the increase in non-specific killing at increased E:T ratios was substantially more pronounced with the SKBR3 cells as compared to the artificial target cells. Therefore, the CAR cytotoxicity result indicated that the artificial target cells SUP-B 15^{HER2} are a substantially improved target as compared to the natural SKBR3 target line.

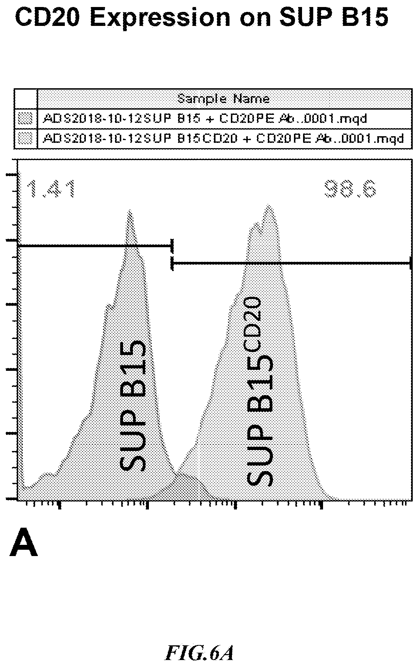

Example 5: CD20 is generally expressed on B cells, however, in certain types of B cell lymphomas and leukemias the CD20 levels are higher than in normal B cells. A stable CD20 expressing SUP-B 15 line was generated by infecting the SUP-B 15 cell with a lentiviral vector carrying the human CD20 cDNA having SEQ ID NO:5 and the puromycin gene. The CD20 expressing cells were selected using puromycin, and surface expression of CD20 was verified by flow cytometry with exemplary results shown in **FIG. 6A****.** As can be seen again, expression of CD20 on the SupB15 cells was strong and in a very large proportion of the cells

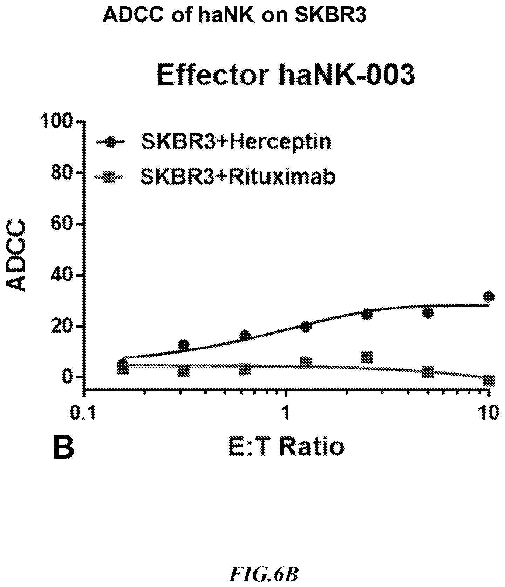

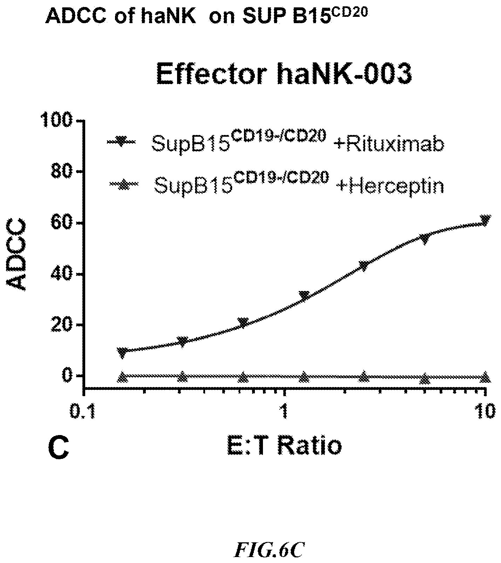

There are very few cell lines which are suitable for ADCC assay *in vitro* and most of them are adherent cell lines. In general, flow cytometry based ADCC assays tends to work better with suspension cells. One aim of the examples presented herein was to use the SUP-B 15^{CD20} cell line to develop flow cytometry based ADCC assays with Rituximab, a monoclonal antibody directed against CD20. Herceptin is another monoclonal antibody that binds selectively to the HER2 protein. Both of antibodies induce cell death via ADCC using NK cells and T cells. For the present example, it should be noted that HER2 is only expressed on the surface of SKBR3 cell line but not on SUP-B15, while CD20 is also not expressed on SKBR3. The SUP-B15^{CD20} line has been validated by comparing ADCC assay along with SKBR3, and exemplary results are depicted in **FIG.6B** and **FIG.6C****.** For the ADCC assay, the inventors have used haNK cells (commercially available from NantKwest^{®}, Inc. 9920 Jefferson Blvd. Culver City, CA 90232), an NK effector cell that expresses a high affinity variant of CD16, and two antibodies, Rituximab and Herceptin. Herceptin is used as a specific antibody for SKBR3 and Rituximab as a specific antibody for SUP-B15^{CD20}, while Herceptin is a control antibody for SUP-B15^{CD20} and Rituximab a control antibody for SKBR3. As can be seen from the data, ADCC for SUP-B15^{CD20} was 60%, and for SKBR3 was 25% at the highest 10:1 ET ratio. It should be readily evident from these data that the artificial target cells showed stronger target specific ADCC as compared to SKBR3 cells.

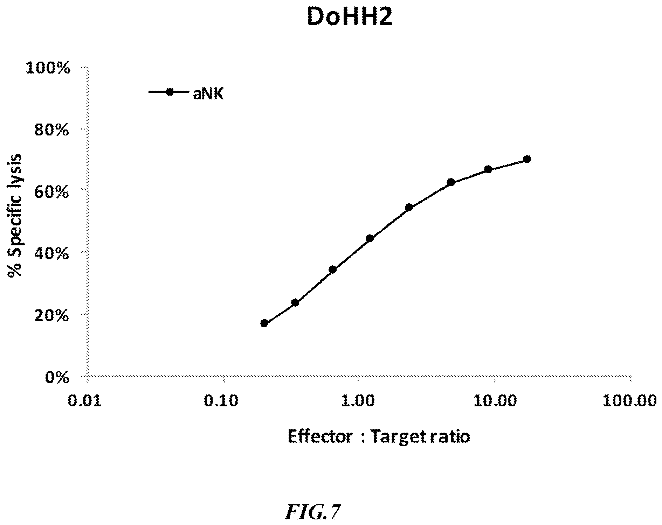

**FIG.7** depicts further comparative data for target expressing cells that are not SUP-B15 and that show high(er) background killing. More specifically, DoHH2 cells are CD19⁺ Non-Hodgkins Lymphoma cell line that is sensitive to aNK cells.

As used herein, the term "administering" a pharmaceutical composition or drug refers to both direct and indirect administration of the pharmaceutical composition or drug, wherein direct administration of the pharmaceutical composition or drug is typically performed by a health care professional (*e.g*., physician, nurse, etc.), and wherein indirect administration includes a step of providing or making available the pharmaceutical composition or drug to the health care professional for direct administration (*e.g*., via injection, infusion, oral delivery, topical delivery, etc.). Most preferably, the cells or exosomes are administered via subcutaneous or subdermal injection. However, in other contemplated aspects, administration may also be intravenous injection. Alternatively, or additionally, antigen presenting cells may be isolated or grown from cells of the patient, infected *in vitro,* and then transfused to the patient. Therefore, it should be appreciated that contemplated systems and methods can be considered a complete drug discovery system (*e.g*., drug discovery, treatment protocol, validation, etc.) for highly personalized cancer treatment.

The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The invention is set out in the appended claims. The use of any and all examples, or exemplary language (*e.g*., "such as") provided with respect to certain embodiments herein is intended merely to better illuminate the full scope of the present disclosure, and does not pose a limitation on the scope of the invention otherwise claimed. No language in the specification should be construed as indicating any non-claimed element essential to the practice of the claimed invention.

It should be apparent to those skilled in the art that many more modifications besides those already described are possible without departing from the full scope of the concepts disclosed herein. The disclosed subject matter, therefore, is not to be restricted except in the scope of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

## Claims

1. A recombinant target cell, comprising: a recombinant nucleic acid comprising a sequence that encodes a transmembrane antigen wherein the sequence is operably coupled to a promoter to express the sequence that encodes the transmembrane antigen;
wherein the cell exhibits equal or less than 20% spontaneous lysis in the presence of a CAR-T or an NK cell at an effector cell to target cell ratio of 1 or less;
wherein the transmembrane antigen is a tumor associated antigen, a tumor specific antigen, or a patient- and tumor specific antigen; and
wherein the recombinant target cell is a SUP-B15 leukaemia cell.

2. The recombinant target cell of claim 1, wherein the transmembrane antigen is an antigen present on a solid tumor or on a liquid tumor.

3. The recombinant target cell of claim 1 or claim 2, wherein the transmembrane antigen is PD-L1, CD33, CD123, HER-2, or CD20.

4. The recombinant target cell of any one of claims 1-3, wherein the recombinant nucleic acid is a recombinant lentiviral expression vector.

5. The recombinant target cell of any one of claims 1-3, wherein the recombinant nucleic acid is a recombinant RNA.

6. The recombinant target cell of claim 1, wherein:
the cell exhibits equal or less than 15% spontaneous lysis in the presence of CAR-T or NK cell at an effector cell to target cell ratio of 10 or less; or
the cell exhibits equal or less than 10% spontaneous lysis in the presence of CAR-T or NK cell at an effector cell to target cell ratio of 1 or less.

7. The recombinant target cell of any one of claims 1-6, wherein the effector cell is a CAR- T cell or an NK cell.

8. A method of quantifying CAR or ADCC mediated cell killing, comprising: providing or generating a recombinant target cell as claimed in any one of claims 1-7; providing or generating a CAR-T or an NK cell; incubating the recombinant target cell and the CAR-T or NK cell at a predetermined effector cell to target cell ratio; and quantifying the ADCC or CAR-mediated cell killing.

9. The method of claim 8 wherein the recombinant target cell exhibits equal or less than 15% spontaneous lysis in the presence of CAR-T or NK cell at an effector cell to target cell ratio of 10 or less or wherein the recombinant target cell exhibits equal or less than 10% spontaneous lysis in the presence of CAR-T or NK cell at an effector cell to target cell ratio of 1 or less.

10. The method of any one of the claims 8-9 wherein the transmembrane antigen expressed by the recombinant target cell is a tumor associated antigen, a tumor specific antigen, or a patient- and tumor specific antigen, optionally wherein the transmembrane antigen is PD-L1, CD33, CD123, HER-2, or CD20.

11. The method of claim 8 wherein:
the step of incubating is performed while the recombinant target cell and the CAR-T or NK cell are in suspension; or
the method is used to screen an antibody library against the transmembrane antigen.

12. The method of any one of claims 8-11 wherein the step of quantifying the ADCC or CAR-mediated cell killing is performed using a flow cytometry-based cytotoxicity or ADCC assay.

## Patentansprüche

1. Rekombinante Zielzelle, umfassend: eine rekombinante Nukleinsäure, die eine Sequenz umfasst, die für ein Transmembranantigen kodiert, wobei die Sequenz an einen Promotor funktionell gekoppelt ist, um die Sequenz zu exprimieren, die für das Transmembranantigen kodiert;
wobei die Zelle in Gegenwart einer CAR-T- oder einer NK-Zelle bei einem Verhältnis von Effektorzellen zu Zielzellen von 1 oder weniger eine spontane Lyse von 20 % oder weniger aufweist;
wobei das Transmembranantigen ein tumorassoziiertes Antigen, ein tumorspezifisches Antigen oder ein patienten- und tumorspezifisches Antigen ist, und
wobei die rekombinante Zielzelle eine SUP-B15-Leukämiezelle ist.

2. Rekombinante Zielzelle nach Anspruch 1, wobei das Transmembranantigen ein auf einem festen Tumor oder auf einem flüssigen Tumor vorhandenes Antigen ist.

3. Rekombinante Zielzelle nach Anspruch 1 oder Anspruch 2, wobei das Transmembranantigen PD-L1, CD33, CD123, HER-2 oder CD20 ist.

4. Rekombinante Zielzelle nach einem der Ansprüche 1 bis 3, wobei die rekombinante Nukleinsäure ein rekombinanter lentiviraler Expressionsvektor ist.

5. Rekombinante Zielzelle nach einem der Ansprüche 1 bis 3, wobei die rekombinante Nukleinsäure eine rekombinante RNA ist.

6. Rekombinante Zielzelle nach Anspruch 1, wobei:
die Zelle in Gegenwart einer CAR-T- oder einer NK-Zelle bei einem Verhältnis von Effektorzellen zu Zielzellen von 10 oder weniger eine spontane Lyse von 15 % oder weniger aufweist, oder
die Zelle in Gegenwart einer CAR-T- oder einer NK-Zelle bei einem Verhältnis von Effektorzellen zu Zielzellen von 1 oder weniger eine spontane Lyse von 10 % oder weniger aufweist.

7. Rekombinante Zielzelle nach einem der Ansprüche 1 bis 6, wobei die Effektorzelle eine CAR-T-Zelle oder eine NK-Zelle ist.

8. Verfahren zum Quantifizieren von CAR- oder ADCC-vermittelter Zellabtötung, umfassend: Bereitstellen oder Erzeugen einer rekombinanten Zielzelle nach einem der Ansprüche 1 bis 7, Bereitstellen oder Erzeugen einer CAR-T- oder einer NK-Zelle, Inkubieren der rekombinanten Zielzelle und der CAR-T- oder NK-Zelle bei einem vorbestimmten Verhältnis von Effektorzellen zu Zielzellen, und Quantifizieren der ADCC- oder CAR-vermittelten Zellabtötung.

9. Verfahren nach Anspruch 8, wobei die rekombinante Zielzelle eine spontane Lyse von gleich oder weniger als 15 % in Gegenwart von CAR-T- oder NK-Zellen bei einem Verhältnis von Effektorzellen zu Zielzellen von 10 oder weniger aufweist oder wobei die rekombinante Zielzelle eine spontane Lyse von gleich oder weniger als 10 % in Gegenwart von CAR-T- oder NK-Zellen bei einem Verhältnis von Effektorzellen zu Zielzellen von 1 oder weniger aufweist.

10. Verfahren nach einem der Ansprüche 8 bis 9, wobei das von der rekombinanten Zielzelle exprimierte Transmembranantigen ein tumorassoziiertes Antigen, ein tumorspezifisches Antigen oder ein patienten- und tumorspezifisches Antigen ist, wobei das Transmembranantigen optional PD-L1, CD33, CD123, HER-2 oder CD20 ist.

11. Verfahren nach Anspruch 8, wobei
der Schritt des Inkubierens durchgeführt wird, während die rekombinante Zielzelle und die CAR-T- oder NK-Zelle in Suspension sind, oder
das Verfahren verwendet wird, um eine Antikörperbibliothek gegen das Transmembranantigen zu screenen.

12. Verfahren nach einem der Ansprüche 8 bis 11, wobei der Schritt des Quantifizierens der ADCC- oder CAR-vermittelten Zellabtötung unter Verwendung eines auf Durchflusszytometrie basierenden Zytotoxizitätstests oder ADCC-Tests durchgeführt wird.

## Revendications

1. Cellule cible recombinante, comprenant : un acide nucléique recombinant comprenant une séquence codant pour un antigène transmembranaire, ladite séquence étant couplée de manière opérationnelle à un promoteur pour exprimer la séquence codant pour l'antigène transmembranaire ;
ladite cellule présentant une lyse spontanée inférieure ou égale à 20 % en présence de cellule CAR-T ou NK à un rapport cellule effectrice/cellule cible de 1 ou moins ;
ledit antigène transmembranaire étant un antigène associé à une tumeur, un antigène spécifique à une tumeur ou un antigène spécifique à un patient et à une tumeur ; et
ladite cellule cible recombinante étant une cellule de leucémie SUP-B15.

2. Cellule cible recombinante de la revendication 1, ledit antigène transmembranaire étant un antigène présent sur une tumeur solide ou sur une tumeur liquide.

3. Cellule cible recombinante de la revendication 1 ou la revendication 2, ledit antigène transmembranaire étant PD-L1, CD33, CD123, HER-2 ou CD20.

4. Cellule cible recombinante de l'une quelconque des revendications 1 à 3, ledit acide nucléique recombinant étant un vecteur d'expression lentiviral recombinant.

5. Cellule cible recombinante de l'une quelconque des revendications 1 à 3, ledit acide nucléique recombinant étant un ARN recombinant.

6. Cellule cible recombinante de la revendication 1,
ladite cellule présentant une lyse spontanée inférieure ou égale à 15 % en présence de cellule CAR-T ou NK à un rapport cellule effectrice/cellule cible de 10 ou moins ; ou
ladite cellule présentant une lyse spontanée inférieure ou égale à 10 % en présence de cellule CAR-T ou NK à un rapport cellule effectrice/cellule cible de 1 ou moins.

7. Cellule cible recombinante de l'une quelconque des revendications 1 à 6, ladite cellule effectrice étant une cellule CAR-T ou une cellule NK.

8. Procédé de quantification de la destruction cellulaire médiée par CAR ou ADCC, comprenant : la fourniture ou la génération d'une cellule cible recombinante selon l'une quelconque des revendications 1 à 7 ; la fourniture ou la génération d'une cellule CAR-T ou NK ; l'incubation de la cellule cible recombinante et de la cellule CAR-T ou NK à un rapport cellule effectrice/cellule cible prédéterminé ; et la quantification de la destruction cellulaire médiée par ADCC ou CAR.

9. Procédé de la revendication 8, ladite cellule cible recombinante présentant une lyse spontanée inférieure ou égale à 15 % en présence de cellule CAR-T ou NK à un rapport cellule effectrice/cellule cible de 10 ou moins ou ladite cellule cible recombinante présentant une lyse spontanée inférieure ou égale à 10 % en présence de cellule CAR-T ou NK à un rapport cellule effectrice/cellule cible de 1 ou moins.

10. Procédé de l'une quelconque des revendications 8 et 9, ledit antigène transmembranaire exprimé par la cellule cible recombinante étant un antigène associé à une tumeur, un antigène spécifique à une tumeur ou un antigène spécifique à un patient et à une tumeur, éventuellement ledit antigène transmembranaire étant PD-L1, CD33, CD123, HER-2 ou CD20.

11. Procédé de la revendication 8,
ladite étape d'incubation étant réalisée alors que la cellule cible recombinante et la cellule CAR-T ou NK sont en suspension ; ou
ledit procédé étant utilisé pour cribler une banque d'anticorps contre l'antigène transmembranaire.

12. Procédé de l'une quelconque des revendications 8 à 11, ladite étape de quantification de la destruction cellulaire médiée par ADCC ou CAR étant réalisée en utilisant un essai de cytotoxicité ou d'ADCC basé sur la cytométrie en flux.
